(19) Europäisches Patentamt
European Patent Office
Office européen des brevets

(11) Veröffentlichungsnummer: **0 219 091**
**B1**

(12) # EUROPÄISCHE PATENTSCHRIFT

(45) Veröffentlichungstag der Patentschrift: **26.04.89**

(51) Int. Cl.⁴: **C07D 309/10**, C07D 309/30, C07D 407/04, C07F 7/18

(21) Anmeldenummer: **86114179.4**

(22) Anmeldetag: **14.10.86**

(54) **Pyranderivate und ein Verfahren zu ihrer Herstellung.**

(30) Priorität: **17.10.85 DE 3536956**

(43) Veröffentlichungstag der Anmeldung: **22.04.87 Patentblatt 87/17**

(45) Bekanntmachung des Hinweises auf die Patenterteilung: **26.04.89 Patentblatt 89/17**

(84) Benannte Vertragsstaaten: **AT BE CH DE FR GB IT LI**

(56) Entgegenhaltungen: **US-A- 2 774 771**

(73) Patentinhaber: **BASF Aktiengesellschaft, Carl-Bosch-Strasse 38, D-6700 Ludwigshafen(DE)**

(72) Erfinder: **Spiegler, Wolfgang, Dr., Westpreussenstrasse 5, D-6520 Worms 27(DE)**
Erfinder: **Goetz, Norbert, Dr., Schoefferstrasse 25, D-6520 Worms 1(DE)**
Erfinder: **Jahn, Dieter, Dr., Burgunder Weg 8, D-6803 Edingen-Neckarhausen(DE)**
Erfinder: **Fischer, Rolf, Dr., Bergstrasse 98, D-6900 Heidelberg(DE)**
Erfinder: **Kropp, Rudolf, Sprottauer Strasse 2, D-6703 Limburgerhof(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die vorliegende Erfindung betrifft neue Pyranderivate sowie ein Verfahren zu ihrer Herstellung, ausgehend von ungesättigten Carbonylverbindungen und Enolethern.

Es ist bekannt, α,β-ungesättigte Aldehyde oder Ketone und Enolether mittels einer Diels-Alder-Reaktion in substituierte Dihydropyrane überzuführen (Chem. Rev. 75, 651 (1975) und Houben-Weyl, Methoden der organischen Chemie 6/4, 355 (1966)). Die bisher dazu als Ausgangsprodukt verwendeten, gegebenenfalls substituieren α,β-ungesättigten Aldehyde oder Ketone weisen aber weder substituierte Methylolgruppen noch derivatisierte Aldehydgruppen als Substituenten an der C–C-Doppelbindung auf. Von diesen Gruppen konnte nämlich erwartet werden, daß sie den drastischen Reaktionsbedingungen der Diels-Alder-Reaktion (Reaktionstemperatur: 180 bis 220°C) nicht standhalten würden, und daß somit Dihydropyranverbindungen, die die genannten Substituenten in 4-Stellung tragen, entweder überhaupt nicht oder allenfalls nur in geringer Ausbeute entstehen würden.

In der US-A 2 774 771 ist weiterhin die Diels-Alder-Reaktion von α,β-ungesättigten Ketonen, die eine derivatisierte Carboxylgruppe an der C–C-Doppelbindung besitzen, mit Enolethern beschrieben, die zu den jeweiligen 6-Alkyldihydropyranen führt. Auch hier war zu erwarten, daß die entsprechenden, in 6-Stellung unsubstituierten Dihydropyrane entweder überhaupt nicht oder nur in schlechter Ausbeute entstehen würden, da die zu ihrer Synthese erforderlichen, durch eine derivatisierte Carboxylgruppe substituierten α,β-ungesättigten Aldehyde unter den genannten Reaktionsbedingungen zu Nebenreaktionen neigen.

Aufgabe der vorliegenden Erfindung war es nun, Pyranderivate mit diesem bisher nicht bekannten Substitutionsmuster bereitzustellen und dabei gleichzeitig einen vorteilhaften Syntheseweg zu beschreiten.

Es wurden neue Pyranderivate der Formeln Ia und Ib gefunden

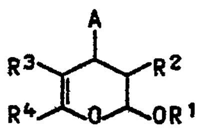
(Ia)
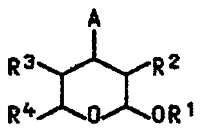
(Ib),

in denen

$R^1$ $C_1$–$C_{18}$-Alkyl oder Phenyl, das gegebenenfalls durch $C_1$–$C_4$-Alkyl, $C_1$–$C_4$-Alkoxy oder Halogen substituiert ist,

$R^2$–$R^4$ Wasserstoff, $C_1$–$C_6$-Alkyl oder Phenyl, das gegebenenfalls durch $C_1$–$C_4$-Alkyl, $C_1$–$C_4$-Alkoxy oder Halogen substituiert ist, und

A den Rest $CH_2$–$OR^5$, in dem $R^5$ für $C_1$–$C_4$-Alkyl oder $C_2$–$C_8$-Alkoxyalkyl, Benzyl, das gegebenenfalls durch $C_1$–$C_4$-Alkyl, $C_1$–$C_4$-Alkoxy oder Halogen im Phenylring substituiert ist, Tetrahydropyran-2-yl, $C_1$–$C_4$-Trialkylsilyl, Formyl, $C_2$–$C_4$-Alkylcarbonyl oder Benzoyl, das gegebenenfalls durch $C_1$–$C_4$-Alkyl, $C_1$–$C_4$-Alkoxy oder Halogen substituiert ist, steht,

den Rest $CH(OR^6)_2$, in dem $R^6$ für $C_1$–$C_4$-Alkyl oder $C_1$–$C_4$-Alkylcarbonyl steht, oder wobei der Rest $CH(OR^6)_2$ für gegebenenfalls $C_1$–$C_4$-alkylsubstituiertes 1,3-Dioxan-2-yl oder gegebenenfalls $C_1$–$C_4$-alkylsubstituiertes 1,3-Dioxolan-2-yl steht, oder

den Rest CO–$OR^7$, in dem $R^7$ für $C_1$–$C_4$-Alkyl oder Phenyl steht, das gegebenenfalls durch $C_1$–$C_4$-Alkyl, $C_1$–$C_4$-Alkoxy oder Halogen substituiert ist, bedeuten,

mit der Maßgabe, daß $R^4$ nicht für $C_1$–$C_4$-Alkyl oder gegebenenfalls substituiertes Phenyl steht, wenn A den Rest CO–$OR^7$ bedeutet.

Die in den Resten $R^1$, $R^2$, $R^3$, $R^4$, $R^5$, $R^6$ und $R^7$ aufgeführten Alkylgruppen können jeweils geradkettig oder verzweigt sein.

$R^1$ in den Formeln Ia und Ib bedeutet z.B. Methyl, Ethyl, Propyl, Isopropyl, Butyl, Isobutyl, tert-Butyl, Pentyl, 2-Methylbutyl, Hexyl, 2-Methylpentyl, Heptyl, Octyl, 2-Ethylhexyl, Isooctyl, Nonyl, Isononyl, Decyl, Isodecyl, 3,5,5,7-Tetramethylnonyl, Isotridecyl, Pentadecyl, Hexadecyl oder Octadecyl. (Die Bezeichnungen Isooctyl, Isononyl, Isodecyl und Isotridecyl sind Trivialbezeichnungen und stammen von den nach der Oxosynthese erhaltenen Alkoholen (vgl. dazu Ullmann, Enzyklopädie der Technischen Chemie, 4. Auflage, Band 7, Seiten 216 und 217 sowie Band 11, Seiten 435 und 436).

$R^1$ bedeutet weiterhin gegebenenfalls substituiertes Phenyl, wie 4-Methylphenyl, 2,4-Dimethylphenyl, 4-Ethylphenyl, 4-Isopropylphenyl, 4-tert-Butylphenyl, 4-Methoxyphenyl, 2-Ethoxyphenyl, 4-Chlorphenyl, 4-Bromphenyl oder 2,4-Dichlorphenyl.

$R^2$, $R^3$ und $R^4$, die gleich oder verschieden sind, bedeuten in den Formeln Ia und Ib unabhängig voneinander beispielsweise Wasserstoff, Methyl, Ethyl, Propyl, Isopropyl, Butyl, Isobutyl, tert-Butyl, Pentyl, 3-Methylbutyl, Hexyl, Phenyl, 4-Methylphenyl, 2,6-Dimethylphenyl, 4-Ethylphenyl, 4-Isopropylphenyl, 4-Methoxyphenyl, 2-Butoxyphenyl, 4-Chlorphenyl oder 2,4-Dichlorphenyl.

A in den Formeln Ia und Ib bedeutet den Rest $CH_2$–$OR^5$, z.B. Methoxymethyl, Ethoxymethyl, Propoxymethyl, Isopropoxymethyl, Butoxymethyl, Isobutoxymethyl, tert-Butoxymethyl, (Methoxymethoxy)methyl,

(1-Isobutoxyethoxy)-methyl, Benzyloxymethyl, 4-Methylbenzyloxymethyl, (4-Methoxybenzyloxy)-methyl, 2-Chlorbenzyloxymethyl, Tetrahydropyran-2-yloxymethyl, Trimethylsilyloxymethyl, Formyloxymethyl, Acetoxymethyl, Propionyloxymethyl, Benzoyloxymethyl, 4-Methylbenzoyloxymethyl oder 4-Chlorbenzoyloxymethyl; den Rest $CH(OR^6)_2$, z.B. Dimethoxymethyl, Diethoxyethyl, Bis(acetoxy)methyl, 1,3-Dioxan-2-yl-, 4-Methyl-1,3-dioxan-2-yl- oder 1,3-Dioxolan-2-yl-; oder den Rest $CO{-}OR^7$, z.B. Methoxycarbonyl, Ethoxycarbonyl, Propoxycarbonyl, Isobutoxycarbonyl, Phenyloxycarbonyl, 4-Methylphenyloxycarbonyl, 4-Ethoxyphenylcarbonyl oder 4-Chlorphenylcarbonyl.

Bevorzugte Pyranderivate der Formel Ia und Ib sind solche, in denen $R^1$ Methyl, Ethyl oder Isobutyl, $R^2$, $R^3$ und $R^4$ gleich oder verschieden sind und unabhängig voneinander jeweils Wasserstoff oder Methyl und A Methoxymethyl, tert-Butoxymethyl, Benzyloxymethyl, Tetrahydropyran-2-yl, Bis(acetoxy)methyl, Dimethoxymethyl, 1,3-Dioxolan-2-yl, 5,5-Dimethyl-1,3-dioxan-2-yl oder Methoxycarbonyl bedeuten.

Bevorzugt sind insbesondere Verbindungen gemäss Anspruch 2 und ganz besonders diejenigen gemäss Anspruch 3.

Die erfindungsgemäßen Pyranderivate der Formel Ia werden vorteilhaft erhalten, wenn man eine ungesättigte Carbonylverbindung der Formel II

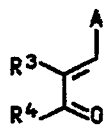 (II)

mit einem Enolether der Formel III

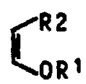 (III)

umsetzt.

Die oben genannten Formeln II und III sollen dabei diejenigen Verbindungen umfassen, in denen der Rest A sowohl cis- als auch trans-ständig zum Rest $R^3$, bzw. der Rest $R^2$ sowohl cis- als auch trans-ständig zum Rest $OR^1$ ist.

Die Umsetzung wird zweckmäßig bei einer Temperatur von 100 bis 300°C, vorzugsweise 150 bis 250°C und einem Druck von 1 bis 1000, vorzugsweise von 70 bis 100 bar, gegebenenfalls in Anwesenheit eines inerten Lösungsmittels und eines inerten Schutzgases vorgenommen.

Für den Fall, daß man die Diels-Alder-Reaktion in Gegenwart eines inerten Lösungsmittels durchführt, kommen beispielsweise Kohlenwasserstoffe, wie Hexan, Cyclohexan, Heptan, Petrolether, Ligroin, Benzol, Toluol oder Xylol; Ether wie Tetrahydrofuran, Tetrahydropyran oder Dioxan; Ketone wie Aceton, Butanon, Methylisopropylketon oder Methylisobutylketon; oder Ester wie Essigsäureethylester sowie deren Gemische in Betracht.

Da es sich bei den als Ausgangsprodukt verwendeten Enolethern der Formel III sowie bei den als Reaktionsprodukt entstehenden Dihydropyranderivaten der Formel Ia, die ebenfalls eine Enoletherstruktur aufweisen, um polymerisierbare Verbindungen handelt, ist es zweckmäßig, die Umsetzung in Gegenwart eines inerten Schutzgases (z.B. Stickstoff, Helium oder Argon) durchzuführen. Weiterhin empfiehlt es sich aus diesem Grunde auch, dem Reaktionsgemisch Inhibitoren, z.B. Hydrochinon, Pyrogallol oder β-Naphthol und Basen, wie Natriumhydroxid, Kaliumhydroxid, Natriumcarbonat, Natriumhydrogencarbonat, Kaliumcarbonat, Calciumcarbonat oder Amine, beispielsweise Triethylamin oder N,N-Dimethylanilin zuzusetzen.

Die Herstellung der Dihydropyrane der Formel Ia nach dem erfindungsgemäßen Verfahren führt man zweckmäßig so durch, daß man ein Gemisch aus ungesättigter Carbonylverbindung der Formel II und Enolether der Formel III in einem Reaktor im genannten Druckbereich auf die Reaktionstemperatur erwärmt. Die Ausgangsprodukte können dabei in stöchiometrischen Mengen eingesetzt werden. Aber auch die Verwendung einer der beiden Komponenten im Überschuß kann vorteilhaft sein, z.B. zur Erhöhung der Reaktionsgeschwindigkeit oder anstelle eines Lösungsmittels.

Die Reaktionsdauer beträgt im allgemeinen 1 bis 4 Stunden, kann aber durch geeignete Maßnahmen auch noch gesenkt werden.

Die Umsetzung kann in kontinuierlicher oder diskontinuierlicher Arbeitsweise durchgeführt werden.

Nach Beendigung der Reaktion kann das Zielprodukt durch Destillation vom Lösungsmittel oder von überschüssiger Ausgangskomponente II oder III abgetrennt und in reiner Form isoliert werden, wobei auch die überschüssigen Ausgangsprodukte II oder III wieder zurückgewonnen werden können. Es ist aber auch möglich, die Dihydropyrane ohne Reinigungsschritt weiter umzusetzen.

Gegebenenfalls kann in einer Folgestufe das entstandene Dihydropyranderivat der Formel Ia mittels Wasserstoff in Gegenwart eines Hydrierkatalysators in ein Tetrahydropyranderivat der Formel Ib übergeführt werden.

Die Hydrierreaktion wird zweckmäßig bei einer Temperatur von –20 bis +300°C, vorzugsweise +10 bis 100°C und einem Druck von 1 bis 300 bar, vorzugsweise 1 bis 50 bar, gegebenenfalls in Anwesenheit eines inerten Lösungsmittels durchgeführt.

Als Hydrierkatalysatoren können die üblichen, an sich bekannten Trägerkatalysatoren oder auch trägerfreie Katalysatoren eingesetzt werden. Vorzugsweise verwendet man solche Katalysatoren, die als aktive Komponente Metalle der Gruppe 8 und 1B des Periodensystems enthalten. Beispielhaft seien die Metalle Kobalt, Nickel, Ruthenium, Iridium, Palladium, Platin oder Kupfer genannt.

Für den Fall, daß man die Hydrierung mit Trägerkatalysatoren durchführt, kann man solche Katalysatoren verwenden, bei denen das jeweilige Metall auf einen inerten Träger aufgebracht worden ist (z.B. durch Tränken mit einer Metallsalzlösung) oder auch gemeinsam mit dem Inertmaterial aus einer vereinigten Salzlösung gefällt worden ist. Als Inertmaterial eignet sich dabei z.B. poröse Kieselsäure, Erdalkalisilikat, Bariumsulfat, Aluminiumoxid oder Kohle. Weiterhin können auch basische Zusätze, beispielsweise Erdalkalioxide, im Katalysator enthalten sein.

Als inerte Lösungsmittel kommen beispielsweise Alkohole, wie Methanol, Ethanol oder Isobutanol; cyclische Ether, wie Tetrahydrofuran oder Tetrahydropyran; Kohlenwasserstoffe, wie Hexan, Cyclohexan, Heptan, Petrolether, Ligroin, Benzol, Toluol oder Xylol; Ester, wie Essigsäureethylester oder Ketone, wie Aceton, Methylisopropylketon oder Methylisobutylketon, sowie deren Gemische, in Betracht.

Die Hydrierung kann sowohl in kontinuierlicher als auch in diskontinuierlicher Arbeitsweise durchgeführt werden.

Die Zielprodukte fallen dabei in hoher Ausbeute und Reinheit an und können nach Abtrennen des Katalysators gegebenenfalls durch Destillation vom Lösungsmittel befreit werden.

Die erfindungsgemäßen Pyranderivate der Formeln Ia und Ib sind wertvolle Zwischenprodukte für die Synthese von Pflanzenschutzmitteln, Pharmazeutika oder Farbstoffen. Insbesondere sind dabei Herbizide auf Cyclohexandionbasis zu nennen, wie sie z.B. in der DE-A 3 121 355 beschrieben sind.

Die Dihydropyranderivate der Formel Ia können dazu nach an sich bekannten Methoden in einer Stufe durch Hydrierung und Hydrogenolyse direkt in solche Tetrahydropyranderivate übergeführt werden, die an Stelle des Reste $OR^1$ ein Wasserstoffatom besitzen. Es ist aber auch möglich, die Pyranderivate der Formel Ib durch hydrogenolytische Abspaltung von $R^1OH$ oder durch Eliminierng von $R^1OH$ und sich anschließende Hydrierung in die genannten Tetrahydropyranderivate umzuwandeln. Diese Produkte können dann, gegebenenfalls nach Überführung der Gruppe A in die jeweils gewünschte Funktionalität (z.B. eine Hydroxymethyl-, Aldehyd- oder Carboxylgruppe), direkt in das Cyclohexansystem eingebaut werden (vgl. dazu z.B. DE-A 3 121 355).

Im Vergleich zu bekannten Syntheseschritten (z.B. J. Chem. Soc. 1930, 2525; Angew. Chem. 86, 742 (1974); J. Am. Chem. Soc. 97, 210 (1975)) bietet das neue Verfahren eine Reihe von Vorteilen. So können mittels des erfindungsgemäßen Verfahrens die neuen Pyranderivate der Formeln Ia und Ib in überraschend hohen Ausbeuten erhalten werden. Weiterhin kann auf die Verwendung von toxischen Substanzen (z.B. Bis(2-chlorethyl)ether) verzichtet werden. Außerdem wird der Cyclisierungsschritt ohne den bisher auftretenden Salzanfall durchgeführt. Schließlich handelt es sich bei den im Verfahren benötigten Einsatzstoffen um leicht zugängliche Produkte.

Die nachfolgenden Beispiele sollen die Erfindung näher erläutern.

Herstellung der Dihydropyrane der Formel Ia

Beispiel 1

Ein Gemisch aus 319 g β-Formylacrylsäuremethylester, 325 g Methylvinylether, 5 g Hydrochinon, 5 g Natriumcarbonat und 600 ml Toluol wurde in einem 2 l-Autoklaven unter einem Stickstoffdruck von 10 bar auf 180°C erwärmt und 3 Stunden bei dieser Temperatur gerührt. Nach dem Abkühlen wurde der Feststoff abgesaugt und das Filtrat destilliert. Man erhielt 439 g (90% d. Th.) 2-Methoxy-3,4-dihydro-2H-pyran-4-carbonsäure-methylester (Verbindung Nr. 1) Siedepunkt: 72°C/2 mbar.

In analoger Weise können die in Tabelle 1 aufgeführten Dihydropyrane erhalten werden.

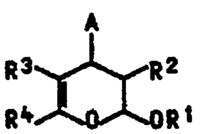

**Tabelle 1**

| Verbindung Nr. | A | $R^1$ | $R^2$ | $R^3$ | $R^4$ | Ausbeute [%] | Siedepunkt [°C/mbar] |
|---|---|---|---|---|---|---|---|
| 2 | $COOCH_3$ | $C_2H_5$ | H | $CH_3$ | H | 74 | 107/18 |
| 3 | $COOC_2H_5$ | iso-$C_4H_9$ | H | $CH_3$ | H | 61 | 134/13 |
| 4 | $COOCH_3$ | $C_2H_5$ | H | H | H | 91 | 83/2,7 |
| 5 | $COOCH_3$ | iso-$C_4H_9$ | H | H | H | 96 | 135/26 |
| 6 | $COOCH_3$ | $C_2H_5$ | $CH_3$ | H | H | 97 | 63/0,3 |
| 7 | $COOC_2H_5$ | $C_2H_5$ | H | H | H | 63 | 55/0,2 |
| 8 | COO-iso-$C_4H_9$ | $C_2H_5$ | H | H | H | 90 | 40/0,1 |
| 9 | $COOCH_3$ | $C_{18}H_{37}$ | H | H | H | 88 | Feststoff |
| 10 | $-CH(OCH_3)_2$ | $CH_3$ | H | H | H | 85 | 60/2,3 |
| 11 | $CH(OCH_3)_2$ | $C_2H_5$ | H | H | H | 90 | 62-65/1,0 |
| 12 | $CH(OCH_3)_2$ | iso-$C_4H_9$ | H | H | H | 84 | 87/2,4 |
| 13 | $CH(OCH_3)_2$ | $C_2H_5$ | $CH_3$ | H | H | 76 | 80-86/0,3 |
| 14 | $CH(OCH_3)_2$ | $C_6H_6$ | H | H | H | 75 | 108-112/0,2 |
| 15 | $CH(OCH_3)_2$ | 4-Cl-$C_6H_5$ | H | H | H | 62 | 135-140/0,1 |
| 16 | $CH(OCH_3)_2$ | $C(CH_3)_3$ | H | H | H | 67 | 72-78/0,4 |
| 17 | $-CH(OOCCH_3)_2$ | $C_2H_5$ | H | $CH_3$ | H | 30 | |
| 18 | $-CH(OC_2H_5)_2$ | $C_2H_5$ | H | H | $CH_3$ | | |
| 19 | 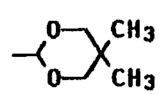 | $CH_3$ | H | H | H | 82 | 83/0,7 |
| 20 | 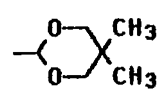 | $C_2H_5$ | H | H | H | | |

Tabelle 1 (Forts.)

| Verbindung Nr. | A | $R^1$ | $R^2$ | $R^3$ | $R^4$ | Ausbeute [%] | Siedepunkt [°C/mbar] |
|---|---|---|---|---|---|---|---|
| 21 | 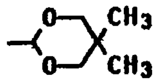 | iso-$C_4H_9$ | H | H | H | 84 | 90-94/0,3 |
| 22 | 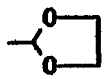 | $CH_3$ | H | H | H | | |
| 23 | 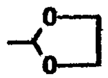 | $C_2H_5$ | H | H | H | 73 | 97/0,4 |
| 24 | 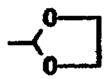 | iso-$C_4H_9$ | H | H | H | | |
| 25 | $-CH(OCH_3)_2$ | $C_{18}H_{37}$ | H | H | H | 73 | 180-186/0,2 |
| 26 | $-CH_2-OCH_3$ | $CH_3$ | H | H | H | | |
| 27 | $-CH_2-OCH_3$ | $C_2H_5$ | H | H | H | | |
| 28 | $-CH_2-OCH_3$ | iso-$C_4H_9$ | H | H | H | | |
| 29 | $-CH_2-O-COCH_3$ | $CH_3$ | H | H | H | | |
| 30 | $-CH_2-O-COCH_3$ | $C_2H_5$ | H | H | H | 75 | 116-124/18 |
| 31 | $-CH_2-O-COCH_3$ | iso-$C_4H_9$ | H | H | H | | |
| 32 | $-CH_2-O-COCH_3$ | $C_{18}H_{37}$ | H | H | H | | |
| 33 | $-CH_2-O-COCH_3$ | $CH_3$ | H | $CH_3$ | H | 78 | 120-125/20 |
| 34 | $-CH_2-O-COCH_3$ | $C_2H_5$ | $CH_3$ | $CH_3$ | H | | |
| 35 | $-CH_2-O-COCH_3$ | $C_2H_5$ | H | $CH_3$ | $CH_3$ | | |
| 36 | $-CH_2-O-COCH_3$ | iso-$C_4H_9$ | H | H | $CH_3$ | | |
| 37 | $-CH_2-O-COC_6H_5$ | iso-$C_4H_9$ | H | H | H | | |

**Tabelle 1** (Forts.)

| Verbindung Nr. | A | $R^1$ | $R^2$ | $R^3$ | $R^4$ | Ausbeute [%] | Siedepunkt [°C/mbar] |
|---|---|---|---|---|---|---|---|
| 38 | $-CH_2-O-C(CH_3)_3$ | $CH_3$ | H | H | H | | |
| 39 | $-CH_2-O-C(CH_3)_3$ | $C_2H_5$ | H | H | H | | |
| 40 | $-CH_2-O-C(CH_3)_3$ | iso-$C_4H_9$ | H | H | H | | |
| 41 | $-CH_2-O-C(CH_3)_3$ | $C_2H_5$ | $CH_3$ | H | H | | |
| 42 | $-CH_2-O-CH_2-C_6H_5$ | $CH_3$ | H | H | H | | |
| 43 | $-CH_2-O-CH_2-C_6H_5$ | $C_2H_5$ | H | H | H | | |
| 44 | $-CH_2-O-CH_2-C_6H_5$ | iso-$C_4H_9$ | H | H | H | | |
| 45 | $-CH_2-O-CH_2-C_6H_5$ | $C_2H_5$ | $CH_3$ | H | H | | |
| 46 | $-CH_2O-$ 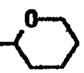 | $CH_3$ | H | H | H | | |
| 47 | $-CH_2O-$ 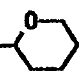 | $C_2H_5$ | H | H | H | 65 | 86-88/0,1 |
| 48 | $-CH_2O-$ 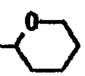 | iso-$C_4H_5$ | H | H | H | | |
| 49 | $-CH_2O-$ 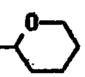 | $C_2H_5$ | $CH_3$ | H | H | | |
| 50 | $-CH_2O-$ 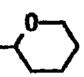 | iso-$C_4H_9$ | H | $CH_3$ | $CH_3$ | | |
| 51 | $-CH_2O-$ 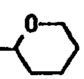 | $C_2H_5$ | H | $CH_3$ | H | | |

**Tabelle 1** (Forts.)

| Verbindung Nr. | A | $R^1$ | $R^2$ | $R^3$ | $R^4$ | Ausbeute [%] | Siedepunkt [°C/mbar] |
|---|---|---|---|---|---|---|---|
| 52 | $-CH_2O-$ 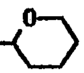 | $C_2H_5$ | H | H | $CH_3$ | | |
| 53 | $-CH_2-O-CH-CH_3$<br>$OCH_3$ | $CH_3$ | H | H | H | | |
| 54 | $-CH_2-O-CH_2$<br>$OCH_3$ | iso-$C_4H_9$ | H | H | H | | |
| 55 | $-CH_2O-CH-CH_3$<br>$O-isoC_4H_9$ | $CH_3$ | H | H | H | | |
| 56 | $-CH_2O-CH-CH_3$<br>$O-isoC_4H_9$ | iso-$C_4H_9$ | H | H | H | | |
| 57 | $-CH_2-O-Si(CH_3)_3$ | $C_2H_5$ | H | H | H | | |

Herstellung der Tetrahydropyrane der Formel Ib

Beispiel 58

In ein Gemisch aus 303 g 2-Ethoxy-3.4-dihydro-2H-pyran-4-aldehyddimethylacetal (Verbindung Nr. 11), 30 g Raney-Nickel und 2 Liter Ethanol wurde bei 25 bis 30°C während 8 Stunden Wasserstoff eingeleitet. Das Reaktionsgemisch wurde dann über Kieselgur abgesaugt und das Filtrat destilliert. Man erhielt 273 g (90% d.Th.) 2-Ethoxytetrahydropyran-4-aldehyd-dimethylacetal (Verbindung Nr. 58) Siedepunkt: 63°C/0,5 mbar.

In analoger Weise können die in Tabelle 2 aufgeführten Tetrahydropyrane erhalten werden.

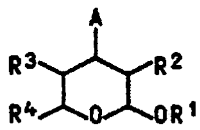

**Tabelle 2**

| Verbindung Nr. | A | $R^1$ | $R^2$ | $R^3$ | $R^4$ | Ausbeute [%] | Siedepunkt [°C/mbar] |
|---|---|---|---|---|---|---|---|
| 59 | $-COOCH_3$ | $CH_3$ | H | H | H | 98 | 75-79/2,0 |
| 60 | $-COOCH_3$ | $C_2H_5$ | H | H | H | 96 | 48-50/0,01 |
| 61 | $-COOC_2H_5$ | $C_2H_5$ | H | H | H | | |
| 62 | $-COO-isoC_4H_9$ | $C_2H_5$ | H | H | H | 86 | 55-60/0,1 |
| 63 | $-CH(OCH_3)_2$ | $CH_3$ | H | H | H | 92 | 117-118/24 |
| 64 | $-CH(OCH_3)_2$ | iso-$C_4H_9$ | H | H | H | 72 | 69-70/0,1 |
| 65 | $-CH(OCH_3)_2$ | $C_{18}H_{37}$ | H | H | H | 90 | Feststoff |
| 66 | $-CH(OCH_3)_2$ | $C_6H_5$ | H | H | H | 83 | 115/0,1 |
| 67 | $-CH(OCH_3)_2$ | $C(CH_3)_3$ | H | H | H | 74 | 60/0,2 |
| 68 | $-CH(OCH_3)_2$ | $C_2H_5$ | $CH_3$ | H | H | | |
| 69 | 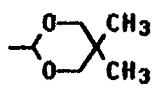 | $CH_3$ | H | H | H | 93 | 69-71/0,2 |
| 70 | 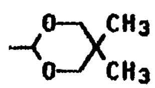 | iso-$C_4H_9$ | H | H | H | | |
| 71 | 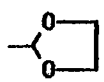 | $CH_3$ | H | H | H | | |
| 72 | 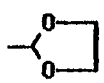 | $C_2H_5$ | H | H | H | 92 | 63-64/0,01 |
| 73 | 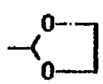 | iso-$C_4H_9$ | H | H | H | | |
| 74 | $-CH_2-OCH_3$ | $CH_3$ | H | H | H | | |

Tabelle 2 (Forts.)

| Verbindung Nr. | A | $R^1$ | $R^2$ | $R^3$ | $R^4$ | Ausbeute [%] | Siedepunkt [°C/mbar] |
|---|---|---|---|---|---|---|---|
| 75 | $-CH_2-OCH_3$ | iso-$C_4H_9$ | H | H | H | | |
| 76 | $-CH_2-O-COCH_3$ | $CH_3$ | H | H | H | | |
| 77 | $-CH_2-O-COCH_3$ | $C_2H_5$ | H | H | H | | |
| 78 | $-CH_2-O-COCH_3$ | iso-$C_4H_9$ | H | H | H | | |
| 79 | $-CH_2OC(CH_3)_3$ | $CH_3$ | H | H | H | | |
| 80 | $-CH_2OC(CH_3)_3$ | $C_2H_5$ | H | H | H | | |
| 81 | $-CH_2OC(CH_3)_3$ | iso-$C_4H_9$ | H | H | H | | |
| 82 | $-CH_2O-$ 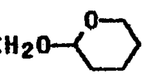 | $CH_3$ | H | H | H | | |
| 83 | $-CH_2O-$ 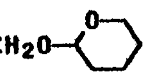 | $C_2H_5$ | H | H | H | | |
| 84 | $-CH_2O-$ 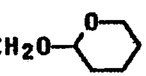 | $C_2H_5$ | H | $CH_3$ | H | | |
| 85 | $-CH_2O-$ 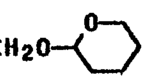 | $C_2H_5$ | H | H | $CH_3$ | | |
| 86 | $-CH_2-O-CH_2$<br>$OCH_3$ | iso-$C_4H_9$ | H | H | H | | |
| 87 | $-CH_2-O-CH-CH_3$<br>$O-isoC_4H_9$ | iso-$C_4H_9$ | H | H | H | | |
| 88 | $-CH_2-O-Si(CH_3)_3$ | $C_2H_5$ | H | H | H | | |

Die folgenden Beispiele Nr. 89 bis 94 sollen die Überführung der erfindungsgemäßen Pyranderivate der Formeln Ia und Ib in solche Tetrahydropyranderivate zeigen, die anstelle der $OR^1$-Gruppe ein Wasserstoffatom besitzen.

Beispiel 89

Auf ein Gemisch aus 248 g 2-Methoxy-3,4-dihydro-2H-pyran-4-carbonsäuremethylester (Verbindung Nr. 1), 5 g Palladium auf Aktivkohle und 1000 ml THF wurden in einem Autoklaven 200 bar Wasserstoff aufgepreßt, dann wurde auf 150°C erwärmt und 20 Stunden bei 150°C und 200 bar Wasserstoff gerührt. Der Reaktionsaustrag wurde über Kieselgur abgesaugt. Destillation des Filtrats ergab 202 g (97% d.Th.) Tetrahydropyran-4-carbonsäuremethylester (Verbindung Nr. 89). Siedepunkt: 77 bis 79°C/15 mbar.

Beispiel 90

10 g 2-Ethoxy-4-(tetrahydropyran-2-yloxymethyl)tetrahydropyran (Verbindung Nr. 83) ergaben unter den in Beispiel 89 beschriebenen Reaktionsbedingungen 3,6 g (75% d.Th.) 4-Hydroxymethyltetrahydropyran (Verbindung Nr. 90). Siedepunkt 92–94°C/12 mbar.

Beispiel 91

Zu einem Gemisch aus 229 g NaOH, 1000 ml Wasser und 700 ml Methanol wurden bei 60°C während 40 Minuten 485 g Tetrahydropyran-4-carbonsäuremethylester (Verbindung Nr. 89) zugetropft, dann wurde 8 Stunden unter Rückfluß gehalten. Nach Destillation des Lösungsmittels gab man 500 ml Wasser zu und extrahierte mit Methyl-tert-butylether. Die Etherphase wurde über $Na_2SO_4$ getrocknet und das Lösungsmittel abdestilliert. Man erhielt als Rückstand 418 g (96% d.Th.) Tetrahydropyranyl-4-carbonsäure (Verbindung Nr. 91). Schmelzpunkt: 84–86°C.

Beispiel 92

Ein Gemisch aus 12,7 g 4-(5,5-Dimethyl-1,3-dioxan-2-yl)-2-methoxy-tetrahydropyran (Verbindung Nr. 69), 0,06 g p-Toluolsulfonsäure und 0,25 g Toluol wurde bei einem Druck von 15 mbar innerhalb von 2 Stunden in einen Kolben getropft, der sich in einem auf 150°C erwärmten Ölbad befand. Gleichzeitig wurden die leichtsiedenden Produkte abdestilliert. Rektifikation des Destillats ergab 6,86 g (62% d.Th.) 4-(5,5-Dimethyl-1,3-dioxan-2-yl)-3,4-dihydro-2H-pyran (Verbindung Nr. 92).
$^1$H-NMR ($CDCl_3$): 0,7 (S, 3H), 1,2 (S, 3H), 1,6–2,1 (M, 3H)
3,2–4,3 (M, 7H), 4,7 (M, 1H), 6,4 (M, 1H) ppm
und 3,3 g (26%) unumgesetztes Ausgangsprodukt.

Beispiel 93

In ein Gemisch aus 4,8 g 4-(5,5-Dimethyl-1,3-dioxan-2-yl)-3,4-dihydro-2H-pyran (Verbindung Nr. 92), 2 g Raney-Nickel und 50 ml Ethanol wurde bei 25°C während 2 Stunden Wasserstoff eingeleitet. Das Reaktionsgemisch wurde über Kieselgur abgesaugt und destilliert. Man erhielt 4,1 g (82% d.Th.) 4-(5,5-Dimethyl-1,3-dioxan-2-yl)-tetrahydropyran (Verbindung Nr. 93).
$^1$H-NMR ($CDCl_3$): 0,7 (S, 3H), 1,2 (S, 3H), 1,4–1,8 (m, 5H),
3,2–4,3 (m, 9H) ppm.

Beispiel 94

Ein Gemisch aus 3,8 g 4-(5,5-Dimethyl-1,3-dioxan-2-yl)-tetrahydropyran (Verbindung Nr. 93) und 40 ml 5%ige Schwefelsäure wurde unter Stickstoff 8 Stunden bei 60°C gerührt. Nach dem Abkühlen wurde mit Methylenchlorid extrahiert, über Natriumsulfat getrocknet und destilliert. Man erhielt 1,3 g (61% d.Th.) 4-Formyltetrahydropyran (Verbindung Nr. 94). Siedepunkt: 74–76°C/25 mbar.

## Patentansprüche für die Vertragsstaaten BE, CH, LI, DE, FR, GB, IT

1. Pyranderivate der Formeln Ia und Ib

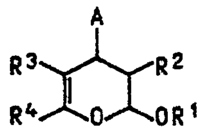 (Ia) 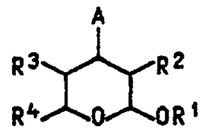 (Ib),

in denen
$R^1$ $C_1$–$C_{18}$-Alkyl oder Phenyl, das gegebenenfalls durch $C_1$–$C_4$-Alkyl, $C_1$–$C_4$-Alkoxy oder Halogen substituiert ist,
$R^2$–$R^4$ Wasserstoff, $C_1$–$C_6$-Alkyl oder Phenyl, das gegebenenfalls durch $C_1$–$C_4$-Alkyl, $C_1$–$C_4$-Alkoxy oder Halogen substituiert ist, und
A den Rest $CH_2$–$OR^5$, in dem $R^5$ für $C_1$–$C_4$-Alkyl oder $C_2$–$C_8$-Alkoxyalkyl, Benzyl, das gegebenenfalls durch $C_1$–$C_4$-Alkyl, $C_1$–$C_4$-Alkoxy oder Halogen im Phenylring substituiert ist, Tetrahydropyran-2-yl, $C_1$–$C_4$-Trialkylsilyl, Formyl, $C_2$–$C_4$-Alkylcarbonyl oder Benzoyl, das gegebenenfalls durch $C_1$–$C_4$-Alkyl, $C_1$–$C_4$-Alkoxy oder Halogen substituiert ist, steht,
den Rest $CH(OR^6)_2$, in dem $R^6$ für $C_1$–$C_4$-Alkyl oder $C_1$–$C_4$-Alkylcarbonyl steht, oder wobei der Rest $CH(OR^6)_2$ für gegebenenfalls $C_1$–$C_4$-alkylsubstituiertes 1,3-Dioxan-2-yl oder gegebenenfalls $C_1$–$C_4$-alkylsubstituiertes 1,3-Dioxolan-2-yl steht, oder
den Rest CO–$OR^7$, in dem $R^7$ für $C_1$–$C_4$-Alkyl oder Phenyl steht, das gegebenenfalls durch $C_1$–$C_4$-Alkyl, $C_1$–$C_4$-Alkoxy oder Halogen substituiert ist, bedeuten,
mit der Maßgabe, daß $R^4$ nicht für $C_1$–$C_4$-Alkyl oder gegebenenfalls substituiertes Phenyl steht, wenn A den Rest CO–$OR^7$ bedeutet.

2. Pyranderivate der Formeln Ia und Ib nach Anspruch 1, in denen
$R^1$ $C_1$–$C_{18}$-Alkyl oder Phenyl, das gegebenenfalls durch $C_1$–$C_4$-Alkyl, $C_1$–$C_4$-Alkoxy oder Halogen substituiert ist,
$R^2$–$R^4$ Wasserstoff, $C_1$–$C_6$-Alkyl oder Phenyl, das gegebenenfalls durch $C_1$–$C_4$-Alkyl, $C_1$–$C_4$-Alkoxy oder Halogen substituiert ist, und
A den Rest $CH_2$–$OR^5$, in dem $R^5$ für $C_1$–$C_4$-Alkyl oder $C_2$–$C_6$-Alkoxyalkyl, Benzyl, das gegebenenfalls durch Methyl, Methoxy oder Chlor im Phenylring substituiert ist, Tetrahydropyran-2-yl, Trimethylsilyl, Formyl, Acetyl, Propionyl oder Benzoyl, das gegebenenfalls durch Methyl oder Chlor substituiert ist, steht,
den Rest $CH(OR^6)_2$, in dem $R^6$ für Methyl, Ethyl oder Acetyl steht, oder wobei der Rest $CH(OR^6)_2$ für gegebenenfalls Methyl substituiertes 1,3-Dioxan-2-yl oder 1,3-Dioxolan-2-yl steht, oder
den Rest CO–$OR^7$, in dem $R^7$ für $C_1$–$C_4$-Alkyl oder Phenyl steht, das gegebenenfalls durch Methyl, Ethoxy oder Chlor substituiert ist, bedeuten,
mit der Maßgabe, daß $R^4$ nicht für $C_1$–$C_4$-Alkyl oder gegebenenfalls substituiertes Phenyl steht, wenn A den Rest CO–$OR^7$ bedeutet.

3. Pyranderivate der Formeln Ia und Ib nach Anspruch 1, in denen
$R^1$ Methyl, Ethyl oder Isobutyl,
$R^2$–$R^4$ Wasserstoff oder Methyl,
A Methoxymethyl, tert.-Butoxymethyl, Benzyloxymethyl, Tetrahydropyran-2-yl, Bis-(acetoxy)methyl, Dimethoxymethyl, 1,3-Dioxolan-2-yl, 5,5-Dimethyl-1,3-dioxan-2-yl oder Methylcarbonyl bedeuten.

4. Verfahren zur Herstellung von Pyranderivaten der Formeln Ia und Ib gemäß Anspruch 1, dadurch gekennzeichnet, daß man eine ungesättigte Carbonylverbindung der Formel II

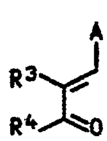 (II)

mit einem Enolether der Formel III

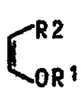 (III)

bei einer Temperatur von 100 bis 300°C und einem Druck von 1 bis 1000 bar, gegebenenfalls in Anwesenheit eines inerten Lösungsmittels und eines inerten Schutzgases umsetzt und gegebenenfalls das entstandene Dihydropyranderivat der Formel Ia mittels Wasserstoff bei einer Temperatur von –20 bis

+300°C und einem Druck von 1 bis 300 bar in Gegenwart eines Hydrierkatalysators in ein Tetrahydropyranderivat der Formel Ib überführt.

**Patentansprüche für den Vertragsstaat AT**

1. Verfahren zur Herstellung von Pyranderivaten der Formeln Ia und Ib

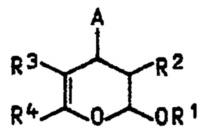 (Ia) 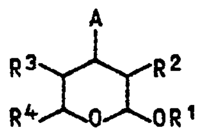 (Ib)

in denen
$R^1$ $C_1$–$C_{18}$-Alkyl oder Phenyl, das gegebenenfalls durch $C_1$–$C_4$-Alkyl, $C_1$–$C_4$-Alkoxy oder Halogen substituiert ist,
$R^2$–$R^4$ Wasserstoff, $C_1$–$C_6$-Alkyl oder Phenyl, das gegebenenfalls durch $C_1$–$C_4$- Alkyl, $C_1$–$C_4$-Alkoxy oder Halogen substituiert ist, und
A den Rest $CH_2$–$OR^5$, in dem $R^5$ für $C_1$–$C_4$-Alkyl oder $C_2$–$C_8$-Alkoxyalkyl, Benzyl, das gegebenenfalls durch $C_1$–$C_4$-Alkyl, $C_1$–$C_4$-Alkoxy oder Halogen im Phenylring substituiert ist, Tetrahydropyran-2-yl, $C_1$–$C_4$-Trialkylsilyl, Formyl, $C_2$–$C_4$-Alkylcarbonyl oder Benzoyl, das gegebenenfalls durch $C_1$–$C_4$-Alkyl, $C_1$–$C_4$-Alkoxy oder Halogen substituiert ist, steht,
den Rest $CH(OR^6)_2$, in dem $R^6$ für $C_1$–$C_4$-Alkyl oder $C_1$–$C_4$-Alkylcarbonyl steht, oder wobei der Rest $CH(OR^6)_2$ für gegebenenfalls $C_1$–$C_4$-alkylsubstituiertes 1,3-Dioxan-2-yl oder gegebenenfalls $C_1$–$C_4$-alkylsubstituiertes 1,3-Dioxolan-2-yl steht, oder
den Rest CO–$OR^7$, in dem $R^7$ für $C_1$–$C_4$-Alkyl oder Phenyl steht, das gegebenenfalls durch $C_1$–$C_4$-Alkyl, $C_1$–$C_4$-Alkoxy oder Halogen substituiert ist, bedeuten,
mit der Maßgabe, daß $R^4$ nicht für $C_1$–$C_4$-Alkyl oder gegebenenfalls substituiertes Phenyl steht, wenn A den Rest CO–$OR^7$ bedeutet, dadurch gekennzeichnet, daß man eine ungesättigte Carbonylverbindung der Formel II

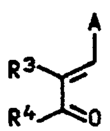 (II)

mit einem Enolether der Formel III

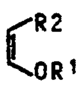 (III)

bei einer Temperatur von 100 bis 300°C und einem Druck von 1 bis 1000 bar, gegebenenfalls in Anwesenheit eines inerten Lösungsmittels und eines inerten Schutzgases umsetzt und gegebenenfalls das entstandene Dihydropyranderivat der Formel Ia mittels Wasserstoff bei einer Temperatur von –20 bis +300°C und einem Druck von 1 bis 300 bar in Gegenwart eines Hydrierkatalysators in ein Tetrahydropyranderivat der Formel Ib überführt.

2. Verfahren zur Herstellung von Pyranderivaten der Formeln Ia und Ib in denen
$R^1$ $C_1$–$C_{18}$-Alkyl oder Phenyl, das gegebenenfalls durch $C_1$–$C_4$-Alkyl, $C_1$–$C_4$-Alkoxy oder Halogen substituiert ist,
$R^2$–$R^4$ Wasserstoff, $C_1$–$C_6$-Alkyl oder Phenyl, das gegebenenfalls durch $C_1$–$C_4$-Alkyl, $C_1$–$C_4$-Alkoxy oder Halogen substituiert ist, und
A den Rest $CH_2$–$OR^5$, in dem $R^5$ für $C_1$–$C_4$-Alkyl oder $C_2$–$C_6$-Alkoxyalkyl, Benzyl, das gegebenenfalls durch Methyl, Methoxy oder Chlor im Phenylring substituiert ist, Tetrahydropyran-2-yl, Trimethylsilyl, Formyl, Acetyl, Propionyl oder Benzoyl, das gegebenenfalls durch Methyl oder Chlor substituiert ist, steht, den Rest $CH(OR^6)_2$, in dem $R^6$ für Methyl, Ethyl oder Acetyl steht, oder wobei der Rest $CH(OR^6)_2$ für gegebenenfalls Methyl substituiertes 1,3-Dioxan-2-yl oder 1,3-Dioxolan-2-yl steht, oder
den Rest CO–$OR^7$, in dem $R^7$ für $C_1$–$C_4$-Alkyl oder Phenyl steht, das gegebenenfalls durch Methyl, Ethoxy oder Chlor substituiert ist, bedeuten,
mit der Maßgabe, daß $R^4$ nicht für $C_1$–$C_4$-Alkyl oder gegebenenfalls substituiertes Phenyl steht, wenn A den Rest CO–$OR^7$ bedeutet, dadurch gekennzeichnet, daß man eine ungesättigte Carbonylverbindung der Formel II

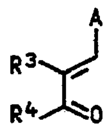

(II)

mit einem Enolether der Formel III

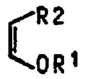

(III)

bei einer Temperatur von 100 bis 300°C und einem Druck von 1 bis 1000 bar, gegebenenfalls in Anwesenheit eines inerten Lösungsmittels und eines inerten Schutzgases umsetzt und gegebenenfalls das entstandene Dihydropyranderivat der Formel Ia mittels Wasserstoff bei einer Temperatur von −20 bis +300°C und einem Druck von 1 bis 300 bar in Gegenwart eines Hydrierkatalysators in ein Tetrahydropyranderivat der Formel Ib überführt.

3. Verfahren zur Herstellung von Pyranderivaten der Formeln Ia und Ib, in denen
$R^1$ Methyl, Ethyl oder Isobutyl,
$R^2$–$R^4$ Wasserstoff oder Methyl,
A Methoxymethyl, tert.-Butoxymethyl, Benzyloxymethyl, Tetrahydropyran-2-yl, Bis-(acetoxy)methyl, Dimethoxymethyl, 1,3-Dioxolan-2-yl, 5,5-Dimethyl-1,3-dioxan-2-yl oder Methylcarbonyl bedeuten, dadurch gekennzeichnet, daß man eine ungesättigte Carbonylverbindung der Formel II

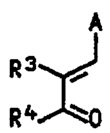

(II)

mit einem Enolether der Formel III

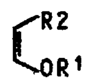

(III)

bei einer Temperatur von 100 bis 300°C und einem Druck von 1 bis 1000 bar, gegebenenfalls in Anwesenheit eines inerten Lösungsmittels und eines inerten Schutzgases umsetzt und gegebenenfalls das entstandene Dihydropyranderivat der Formel Ia mittels Wasserstoff bei einer Temperatur von −20 bis +300°C und einem Druck von 1 bis 300 bar in Gegenwart eines Hydrierkatalysators in ein Tetrahydropyranderivat der Formel Ib überführt.

## Claims for the Contracting States BE, CH, LI, DE, FR, GB, IT

1. A pyran derivative of the formula Ia or Ib

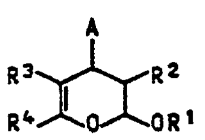

(Ia)

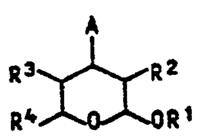

(Ib)

where $R^1$ is $C_1$–$C_{18}$-alkyl or phenyl which is unsubstituted or substituted by $C_1$–$C_4$-alkyl, $C_1$–$C_4$-alkoxy or halogen, $R^2$, $R^3$ and $R^4$ are each hydrogen, $C_1$–$C_6$-alkyl or phenyl which is unsubstituted or substituted by $C_1$–$C_4$-alkyl, $C_1$–$C_4$-alkoxy or halogen, and A is a radical $CH_2$–$OR^5$, where $R_5$ is $C_1$–$C_4$-alkyl or $C_2$–$C_8$-alkoxyalkyl or is benzyl which is unsubstituted or substituted in the phenyl ring by $C_1$–$C_4$-alkyl, $C_1$–$C_4$-alkoxy or halogen, or is tetrahydropyran-2-yl, $C_1$–$C_4$-trialkylsilyl, formyl, $C_2$–$C_4$-alkylcarbonyl or benzoyl which is unsubstituted or substituted by $C_1$–$C_4$-alkyl, $C_1$–$C_4$-alkoxy or halogen, or A is a radical $CH(OR^6)_2$, where $R^6$ is $C_1$–$C_4$-alkyl or $C_1$–$C_4$-alkyl-carbonyl, or a radical $CH(OR^6)_2$ which is unsubstituted or $C_1$–$C_4$-alkyl-substituted 1,3-dioxan-2-yl or unsubstituted or $C_1$–$C_4$-alkyl-substituted 1,3-dioxolan-2-yl, or A is a radical $CO$–$OR^7$, where $R^7$ is $C_1$–$C_4$-alkyl or phenyl which is unsubstituted or substituted by $C_1$–$C_4$-alkyl, $C_1$–$C_4$-alkoxy or halogen, with the proviso that $R^4$ is not $C_1$–$C_4$-alkyl or unsubstituted or substituted phenyl when A is the radical $CO$–$OR^7$.

2. A pyran derivative of the formula Ia or Ib as claimed in claim 1, wherein $R^1$ is $C_1$–$C_{18}$-alkyl or phenyl which is unsubstituted or substituted by $C_1$–$C_4$-alkyl, $C_1$–$C_4$-alkoxy or halogen, $R^2$, $R^3$ and $R^4$ are each hydrogen, $C_1$–$C_6$-alkyl or phenyl which is unsubstituted or substituted by $C_1$–$C_4$-alkyl, $C_1$–$C_4$-alkoxy or halogen, and A is a radical $CH_2$–$OR^5$, where $R^5$ is $C_1$–$C_4$-alkyl, $C_2$–$C_6$-alkoxyalkyl or benzyl which is unsubstituted or substituted in the phenyl ring by methyl, methoxy or chlorine, or is tetrahydropyran-2-yl, trimethylsilyl, formyl, acetyl, propionyl or benzoyl which is unsubstituted or substituted by methyl or chlorine, or A is a radical $CH(OR^6)_2$, where $R^6$ is methyl, ethyl or acetyl, or a radical $CH(OR^6)_2$ which is unsubstituted or methyl-substituted 1,3-dioxan-2-yl or 1,3-dioxolan-2-yl, or A is a radical CO–$OR^7$, where $R^7$ is $C_1$–$C_4$-alkyl or phenyl which is unsubstituted or substituted by methyl, ethoxy or chlorine, with the proviso that $R^4$ is not $C_1$–$C_4$-alkyl or unsubstituted or substituted phenyl when A is CO–$OR^7$.

3. A pyran derivative of the formula Ia or Ib as claimed in claim 1, wherein $R^1$ is methyl, ethyl or isobutyl, $R^2$, $R^3$ and $R^4$ are each hydrogen or methyl and A is methoxymethyl, tert-butoxymethyl, benzyloxymethyl, tetrahydropyran-2-yl, bisacetoxymethyl, dimethoxymethyl, 1,3-dioxolan-2-yl, 5,5-dimethyl-1,3-dioxan-2-yl or methyl-carbonyl.

4. A process for the preparation of a pyran derivative of the formula Ia or Ib as claimed in claim 1, wherein an unsaturated carbonyl compound of the formula II

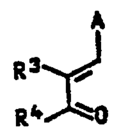

(II)

is reacted with an enol ether of the formula III

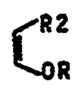

(III)

at from 100 to 300°C under from 1 to 1000 bar in the presence or absence or an inert solvent and of an inert protective gas, and, if desired, the resulting dihydropyran derivative of the formula Ia is converted to a tetrahydropyran derivative of the formula Ib by means of hydrogen at from –20 to +300°C and under from 1 to 300 bar in the presence of a hydrogenation catalyst.

**Claims for the Contracting State: AT**

1. A process for the preparation of a pyran derivative of the formula Ia or Ib

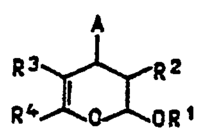

(Ia)

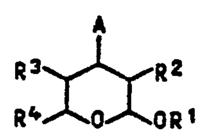

(Ib)

where $R^1$ is $C_1$–$C_{18}$-alkyl or phenyl which is unsubstituted or substituted by $C_1$–$C_4$-alkyl, $C_1$–$C_4$-alkoxy or halogen, $R^2$, $R^3$ and $R^4$ are each hydrogen, $C_1$–$C_6$-alkyl or phenyl which is unsubstituted or substituted by $C_1$–$C_4$-alkyl, $C_1$–$C_4$-alkoxy or halogen, and A is a radical $CH_2$–$OR^5$, where $R^5$ is $C_1$–$C_4$-alkyl or $C_2$–$C_8$-alkoxyalkyl or is benzyl which is unsubstituted or substituted in the phenyl ring by $C_1$–$C_4$-alkyl, $C_1$–$C_4$-alkoxy or halogen, or is tetrahydropyran-2-yl, $C_1$–$C_4$-trialkylsilyl, formyl, $C_2$–$C_4$-alkylcarbonyl or benzoyl which is unsubstituted or substituted by $C_1$–$C_4$-alkyl, $C_1$–$C_4$-alkoxy or halogen, or A is a radical $CH(OR^6)_2$, where $R^6$ is $C_1$–$C_4$-alkyl or $C_1$–$C_4$-alkyl-carbonyl, or a radical $CH(OR^6)_2$ which is unsubstituted or $C_1$–$C_4$-alkyl-substituted 1,3-dioxan-2-yl or unsubstituted or $C_1$–$C_4$-alkyl-substituted 1,3-dioxolan-2-yl, or A is a radical CO–$OR^7$, where $R^7$ is $C_1$–$C_4$-alkyl or phenyl which is unsubstituted or substituted by $C_1$–$C_4$-alkyl, $C_1$–$C_4$-alkoxy or halogen, with the proviso that $R^4$ is not $C_1$–$C_4$-alkyl or unsubstituted or substituted phenyl when A is the radical CO–$OR^7$, wherein an unsaturated carbonyl compound of the formula II

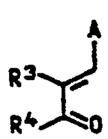

(II)

is reacted with a enol ether of the formula III

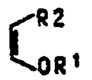

(III)

at from 100 to 300°C under from 1 to 1000 bar in the presence or absence of an inert solvent and of an inert protective gas, and, if desired, the resulting dihydropyran derivative of the formula Ia is converted to a tetrahydropyran derivative of the formula Ib by means of hydrogen at from –20 to +300°C and under from 1 to 300 bar in the presence of a hydrogenation catalyst.

2. A process for the preparation of a pyran derivative of the formula Ia or Ib where $R^1$ is $C_1$–$C_{18}$-alkyl or phenyl which is unsubstituted or substituted by $C_1$–$C_4$-alkyl, $C_1$–$C_4$-alkyoxy or halogen, $R^2$, $R^3$ and $R^4$ are each hydrogen, $C_1$–$C_6$-alkyl or phenyl which is unsubstituted or substituted by $C_1$–$C_4$-alkyl, $C_1$–$C_4$-alkoxy or halogen, and A is a radical $CH_2$–$OR^5$, where $R^5$ is $C_1$–$C_4$-alkyl, $C_2$–$C_6$-alkoxyalkyl or benzyl which is unsubstituted or substituted in the phenyl ring by methyl, methoxy or chlorine, or is tetrahydropyran-2-yl, trimethylsilyl, formyl, acetyl, propionyl or benzoyl which is unsubstituted or substituted by methyl or chlorine, or A is a radical $CH(OR^6)_2$, where $R^6$ is methyl, ethyl or acetyl, or a radical $CH(OR^6)_2$ which is unsubstituted or methyl-substituted 1,3-dioxan-2-yl or 1,3-dioxolan-2-yl, or A is a radical CO–$OR^7$, where $R^7$ is $C_1$–$C_4$-alkyl or phenyl which is unsubstituted or substituted by methyl, ethoxy or chlorine, with the proviso that $R_4$ is not $C_1$–$C_4$-alkyl or unsubstituted or substituted phenyl when A is CO–$OR^7$ wherein an unsaturated carbonyl compound of the formula II

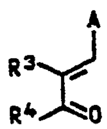

(II)

is reacted with an enol ether of the formula III

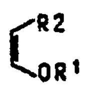

(III)

at from 100 to 300°C under from 1 to 1000 bar in the presence or absence of an inert solvent and of an inert protective gas, and, if desired, the resulting dihydropyran derivative of the formula Ia is converted to a tetrahydropyran derivative of the formula Ib by means of hydrogen at from –20 to +300°C and under from 1 to 300 bar in the presence of a hydrogenation catalyst.

3. A process for the preparation of a pyran derivative of the formula Ia or Ib where $R^1$ is methyl, ethyl or isobutyl, $R^2$, $R^3$ and $R^4$ are each hydrogen or methyl and A is methoxymethyl, tert-butoxymethyl, benzyloxymethyl, tetrahydropyran-2-yl, bisacetoxymethyl, dimethoxymethyl, 1,3-dioxolan-2-yl, 5,5-dimethyl-1,3-dioxan-2-yl or methyl-carbonyl, wherein an unsaturated carbonyl compound of the formula II

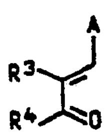

(II)

is reacted with an enol ether of the formula III

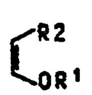

(III)

at from 100 to 300° under from 1 to 1000 bar in the presence or absence of an inert solvent and of an inert protective gas, and, if desired, the resulting dihydropyran derivative of the formula Ia is converted to a tetrahydropyran derivative of the formula Ib by means of hydrogen at from –20 to +300°C and under from 1 to 300 bar in the presence of a hydrogenation catalyst.

**Revendications pour les Etats contractants BE, CH, LI, DE, FR, GB, IT**

1. Dérivés de pyranne de formules Ia et Ib

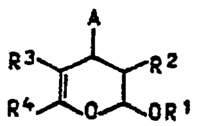 (Ia) 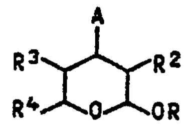 (Ib)

dans lesquelles
$R^1$ est un groupement alkyle en $C_1$–$C_{18}$ ou phényle, (qui est éventuellement substitué par des radicaux alkyle en $C_1$–$C_4$, alcoxy en $C_1$–$C_4$ ou halogène),
$R_2$ à $R_4$ sont des atomes d'hydrogène ou des groupements alkyle en $C_1$–$C_6$ ou phényle (qui est éventuellement substitué par des radicaux alkyle en $C_1$–$C_4$, alcoxy en $C_1$–$C_4$ ou halogène), et
A représente le reste $CH_2$–$OR_5$ où $R^5$ est mis pour un groupement alkyle en $C_1$–$C_4$ ou alcoxyalkyle en $C_2$–$C_8$, benzyle (qui est éventuellement substitué sur le noyau phényle par des radicaux alkle en $C_1$–$C_4$, alcoxy en $C_1$–$C_4$ ou halogène) tétrahydropyranne-2-yle, tri(alkyl en $C_1$–$C_4$)silyle, formyle, alkylcarbonyle en $C_2$–$C_4$ ou benzoyle (qui est éventuellement substitué par des radicaux alkyle en $C_1$–$C_4$, alcoxy en $C_1$–$C_4$ ou halogène)
le reste $CH(OR^6)_2$ où $R^6$ est mis pour un groupement alkyle en $C_1$–$C_4$ ou (alkyl en $C_1$–$C_4$)-carbonyle ou bien le reste $CH(OR^6)_2$ est mis pour un reste 1,3-dioxanne-2-yle éventuellement substitué par un groupement alkyle en $C_1$–$C_4$, ou 1,3-dioxolanne-2-yle éventuellement substitué par un groupement alkyle en $C_1$–$C_4$, ou
le reste CO–$OR^7$ où $R^7$ est mis pour un groupement alkyle en $C_1$–$C_4$ ou phényle qui est éventuellement substitué par des radicaux alkyle en $C_1$–$C_4$, alcoxy en $C_1$–$C_4$ ou halogène,
à la condition que $R^4$ ne représente pas un groupement alkyle en $C_1$–$C_4$ ou phényle éventuellement substitué quand à représente le reste CO–$OR^7$.

2. Dérivés de pyranne de formules Ia et Ib selon la revendication 1, dans lesquelles
$R^1$ est un groupement alkyle en $C_1$–$C_{18}$ ou phényle qui est éventuellement substitué par des radicaux alkyle en $C_1$–$C_4$, alcoxy en $C_1$–$C_4$ ou halogène,
$R^2$ à $R^4$ sont des atomes d'hydrogène ou des groupements alkyle en $C_1$–$C_6$ ou phényle (qui est éventuellement substitué par des radicaux alkyle en $C_1$–$C_4$, alcoxy en $C_1$–$C_4$ ou halogène), et A représente le reste $CH_2$–$OR^5$ où $R^5$ est mis pour un groupement alkyle en $C_1$–$C_4$ ou alcoxyalkyle en $C_2$–$C_6$, benzyle (qui est éventuellement substitué sur le noyau phényle par des radicaux méthyle, méthoxy ou chloro), tétrahydropyranne-2-yle, triméthylsilyle, formyle, acétyle, propionyle ou benzoyle (qui est éventuellement substitué par des radicaux méthyle ou chloro),
le reste $CH(OR^6)_2$ où $R^6$ est mis pour un groupement méthyle, éthyle ou acétyle, ou le reste $CH(OR^6)_2$ est mis pour un groupement 1,3-dioxanne-2-yle ou 1,3-dioxolanne-2-yle éventuellement substitué par des radicaux méthyle,
ou le reste CO–$OR^7$ où $R^7$ est mis pour un groupement alkyle en $C_1$–$C_4$ ou phényle (qui est éventuellement substitué par des radicaux méthyle, éthoxy ou chloro), à la condition que $R^4$ ne représente pas un groupement alkyle en $C_1$–$C_4$ ou phényle éventuellement substitué quand A représente le reste CO–$OR^7$.

3. Dérivés de pyranne de formules Ia et Ib selon la revendication 1, dans lesquelles
$R^1$ est un groupement méthyle, éthyle ou isobutyle, $R^2$ à $R^4$ sont des atomes d'hydrogène ou des groupements méthyle,
A représente un groupement méthoxyméthyle, tert.–butoxyméthyle, benzyloxyméthyle, tétrahydropyranne-2-yle, bis-(acétoxy)méthyle, diméthoxyméthyle, 1,3-dioxolanne-2-yle, 5,5-diméthyl-1,3-dioxanne-2-yle ou méthylcarbonyle.

4. Procédé de préparation de dérivés de pyranne de formules Ia et Ib selon la revendication 1, caractérisé en ce qu'on fait réagir un composé carbonylé insaturé de formule II

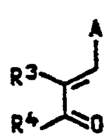 (II)

avec un énol-éther de formule III

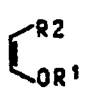 (III)

à une température de 100 à 300°C et sous une pression de 1 à 1000 bars, éventuellement en présence d'un solvant inerte et d'un gaz protecteur inerte, et on transforme éventuellement le dérivé de dihydropyranne obtenu de formule Ia en dérivé de tétrahydropyranne de formule Ib à l'aide d'hydrogène à une température de −20 à +300°C et sous une pression de 1 à 300 bars en présence d'un catalyseur d'hydrogénation.

**Revendications pour l'Etat contractant AT**

1. Procédé de préparation de dérivés de pyranne de formules Ia et Ib

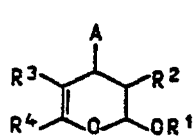
(Ia)
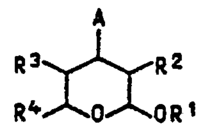
(Ib)

dans lesquelles
$R^1$ est un groupement alkyle en $C_1$–$C_{18}$ ou phényle, (qui est éventuellement substitué par des radicaux alkyle en $C_1$–$C_4$, alcoxy en $C_1$–$C_4$ ou halogène),
$R^2$ à $R^4$ sont des atomes d'hydrogène ou des groupements alkyle en $C_1$–$C_6$ ou phényle (qui est éventuellement substitué par des radicaux alkyle en $C_1$–$C_4$, alcoxy en $C_1$–$C_4$ ou halogène), et
A représente
le reste $CH_2$–$OR^5$ où $R^5$ est mis pour un groupement alkyle en $C_1$–$C_4$ ou alcoxyalkyle en $C_2$–$C_8$, benzyle (qui est éventuellement substitué sur le noyau phényle par des radicaux alkyle en $C_1$–$C_4$, alcoxy en $C_1$–$C_4$ ou halogène) tétrahydropyranne-2-yle, tri(alkyl en $C_1$–$C_4$)silyle, formyle, alkylcarbonyle en $C_2$–$C_4$ ou benzoyle (qui est éventuellement substitué par des radicaux alkyle en $C_1$–$C_4$, alcoxy en $C_1$–$C_4$ ou halogène)
le reste $CH(OR^6)_2$ où $R^6$ est mis pour un groupement alkyle en $C_1$–$C_4$ ou (alkyl en $C_1$–$C_4$)-carbonyle ou bien le reste $CH(OR^6)_2$ est mis pour un reste 1,3-dioxanne-2-yle éventuellement substitué par un groupement alkyle en $C_1$–$C_4$, ou 1,3-dioxolanne-2-yle éventuellement substitué par un groupement alkyle en $C_1$–$C_4$, ou
le reste CO–$OR^7$ où $R^7$ est mis pour un groupement alkyle en $C_1$–$C_4$ ou phényle qui est éventuellement substitué par des radicaux alkyle en $C_1$–$C_4$, alcoxy en $C_1$–$C_4$ ou halogène,
à la condition que $R^4$ ne représente pas un groupement alkyle en $C_1$–$C_4$ ou phényle éventuellement substitué quand A représente le reste CO–$OR^7$,
caractérisé en ce qu'on fait réagir un composé carbonylé insaturé de formule II

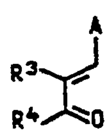
(II)

avec un énol-éther de formule III

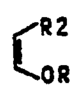
(III)

à une température de 100 à 300°C et sous une pression de 1 à 1000 bars, éventuellement en présence d'un solvant inerte et d'un gaz protecteur inerte, et on transforme éventuellement le dérivé de dihydropyranne obtenu de formule Ia en dérivé de tétrahydropyranne de formule Ib à l'aide d'hydrogène à une température de −20 à +300°C et sous une pression de 1 à 300 bars en présence d'un catalyseur d'hydrogénation.

2. Procédé de préparation de dérivés de pyranne de formules Ia et Ib, dans lesquelles
$R^1$ est un groupement alkyle en $C_1$–$C_{18}$ ou phényle qui est éventuellement substitué par des radicaux alkyle en $C_1$–$C_4$, alcoxy en $C_1$–$C_4$ ou halogène,
$R^2$ à $R^4$ sont des atomes d'hydrogène ou des groupements alkyle en $C_1$–$C_6$ ou phényle (qui est éventuellement substitué par des radicaux alkyle en $C_1$–$C_4$, alcoxy en $C_1$–$C_4$ ou halogène), et
A représente
le reste $CH_2$–$OR^5$ où $R^5$ est mis pour un groupement alkyle en $C_1$–$C_4$ ou alcoxyalkyle en $C_2$–$C_6$, benzyle (qui est éventuellement substitué sur le noyau phényle par des radicaux méthyle, méthoxy ou chloro), tétrahydropyranne-2-yle, triméthylsilyle, formyle, acétyle, propionyle ou benzoyle (qui est éventuellement substitué par des radicaux méthyle ou chloro),

le reste $CH(OR^6)_2$ où $R^6$ est mis pour un groupement méthyle, éthyle ou acétyle, ou le reste $CH(OR^6)_2$ est mis pour un groupement 1,3-dioxanne-2-yle ou 1,3-dioxolanne-2-yle éventuellement substitué par des radicaux méthyle,
ou
le reste $CO–OR^7$ où $R^7$ est mis pour un groupement alkyle en $C_1–C_4$ ou phényle (qui est éventuellement substitué par des radicaux méthyle, éthoxy ou chloro), à la condition que $R^4$ ne représente pas un groupement alkyle en $C_1–C_4$ ou phényle éventuellement substitué quand A représente le reste $CO–OR^7$,
caractérisé en ce qu'on fait réagir un composé carbonylé insaturé de formule II

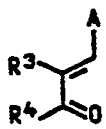

(II)

avec un énol-éther de formule III

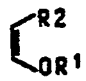

(III)

à une température de 100 à 300°C et sous une pression de 1 à 1000 bars, éventuellement en présence d'un solvant inerte et d'un gaz protecteur inerte, et on transforme éventuellement le dérivé de dihydropyranne obtenu de formule Ia en dérivé de tétrahydropyranne de formule Ib à l'aide d'hydrogène à une température de –20 à +300°C et sous une pression de 1 à 300 bars en présence d'un catalyseur d'hydrogénation.

3. Procédé de préparation de dérivés de pyranne de formules Ia et Ib, dans lesquels
$R^1$ est un groupement méthyle, éthyle ou isobutyle,
$R^2$ à $R^4$ sont des atomes d'hydrogène ou des groupements méthyle,
A représente un groupement méthoxyméthyle, tert.-butoxyméthyle, benzyloxyméthyle, tétrahydropyranne-2-yle, bis-(acétoxy)méthyle, diméthoxyméthyle, 1,3-dioxolanne-2-yle, 5,5-diméthyl-1,3-dioxanne-2-yle ou méthylcarbonyle,
caractérisé en ce qu'on fait réagir un composé carbonylé insaturé de formule II

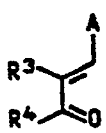

(II)

avec un énol-éther de formule III

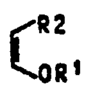

(III)

à une tempàrature de 100 à 300°C et sous une pression de 1 à 1000 bars, éventuellement en présence d'un solvant inerte et d'un gaz protecteur inerte, et on transforme éventuellement le dérivé de dihydropyranne obtenu de formule Ia en dérivé de tétrahydropyranne de formule Ib à l'aide d'hydrogène à une température de –20 à +300°C et sous une pression de 1 à 300 bars en présence d'un catalyseur d'hydrogénation.